# EUROPEAN PATENT SPECIFICATION

(11) **EP 3 586 799 B1**
(45) Date of publication and mention of the grant of the patent: **07.04.2021**
(21) Application number: 19168076.8
(22) Date of filing: 09.04.2019
(51) Int. Cl.: A61F 2/28

(54) **BONE PROSTHESIS**
KNOCHENPROTHESE
PROTHÈSE OSSEUSE

(30) Priority: 21.06.2018 IT 201800006534
(43) Date of publication of application: 01.01.2020
(73) Proprietor: Adler Ortho S.p.a., 20032 Cormano (IT); Azienda Ospedaliero-Universitaria Careggi, 50134 Firenze (IT)
(72) Inventor: BELTRAMI, Giovanni, 50126 FIRENZE (IT)
(74) Representative: Modiano, Micaela Nadia

(56) References cited:
- WO-A1-98/29058
- WO-A1-03/005938
- CN-U- 202 654 268
- DE-U1- 9 115 341
- FR-A1- 2 819 715
- US-A1- 2005 112 397

## Description

The present invention relates to a prosthesis, in particular of the type made of titanium and/or alloys thereof, which is designed to replace bone portions that have been sectioned during an osteotomy operation.

In this technical sector, it is known that prostheses made of titanium and/or alloys thereof are the ones that are best adapted to be customized (custom made) for the specific patient, so as to reconstruct even relatively extensive portions of bone tissue and to perfectly match with the osteotomy lines.

A known problem with these prostheses is long-term duration: such prostheses are in fact sometimes subject to mechanical breakage and to poor integration with the existing bone tissue at the osteotomy planes.

Furthermore, even if the prosthesis integrates with the bone tissue, this is usually superficial and affects only a few millimeters of thickness, so that it is difficult to make a major modification to the structural characteristics of the prosthesis.

In the long term, these limitations can easily compromise the functionality of the prosthesis, especially if the prosthesis is subjected to high loads, which for example is the case with a pelvis prosthesis (also known as a hemipelvic prosthesis) Document WO 03/005938 discloses a prosthesis according to the preamble of claim 1 of the present invention.

The aim of the present invention consists of providing a prosthesis that solves the above technical problem, eliminates the drawbacks and overcomes the limitations of the known art, while making it possible to maintain the functionality of the prosthesis in the long term and to improve the structural characteristics of the overall bone/prosthesis structure.

Within this aim, an object of the present invention is to provide a prosthesis that enables an excellent integration with the existing bone tissue at least at the osteotomy planes.

Another object of the invention consists of providing a prosthesis that is capable of offering the widest guarantees of reliability and safety in use.

Another object of the invention consists of providing a prosthesis that is easy to implement and economically competitive when compared to the known art.

This aim and these and other objects which will become better apparent hereinafter are achieved by a bone prosthesis comprising a prosthesis body which is adapted to be coupled to a bone and which comprises, for this purpose, at least one fixing wing which protrudes externally from said body for screw fixing on said bone, wherein at least one channel is open onto the prosthesis body and is designed to receive a graft, preferably an autologous vascularized bone tissue and/or bank bone and/or bone substitutes and/or stem cells and/or fasciae and/or muscles and/or a device with an antimicrobial capability or function, said channel extending up to perimetric edges of the body.

Further characteristics and advantages of the invention will become better apparent from the detailed description of a preferred, but not exclusive, embodiment of a prosthesis, which is illustrated by way of non-limiting example with the aid of the accompanying drawings wherein:

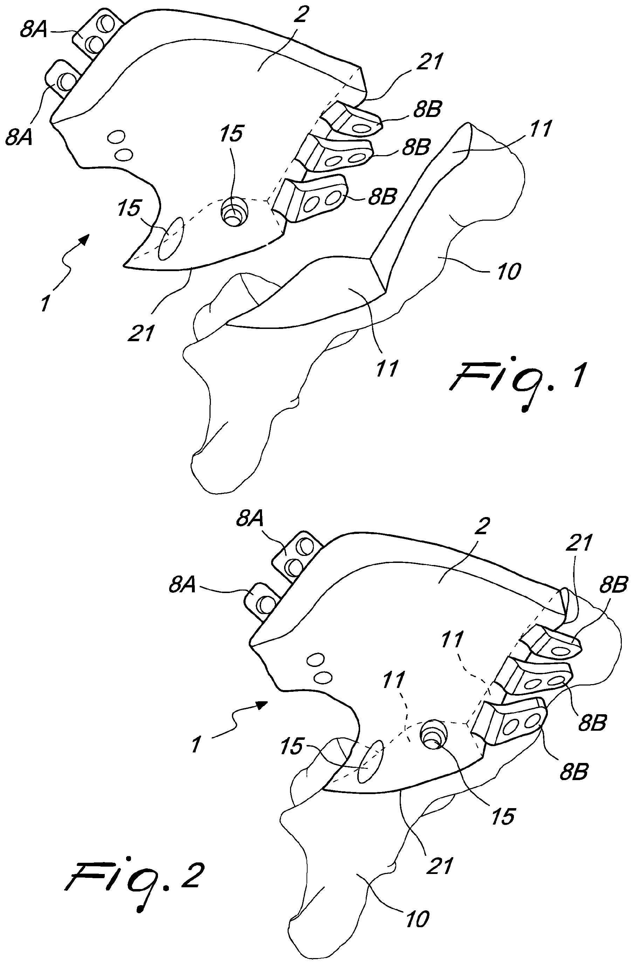

Figure 1 is an exploded perspective view, seen from a first angle of view, of an embodiment of a prosthesis, according to the invention;
Figure 2 is a perspective view of the prosthesis in Figure 1 positioned on the bone of the pelvis;

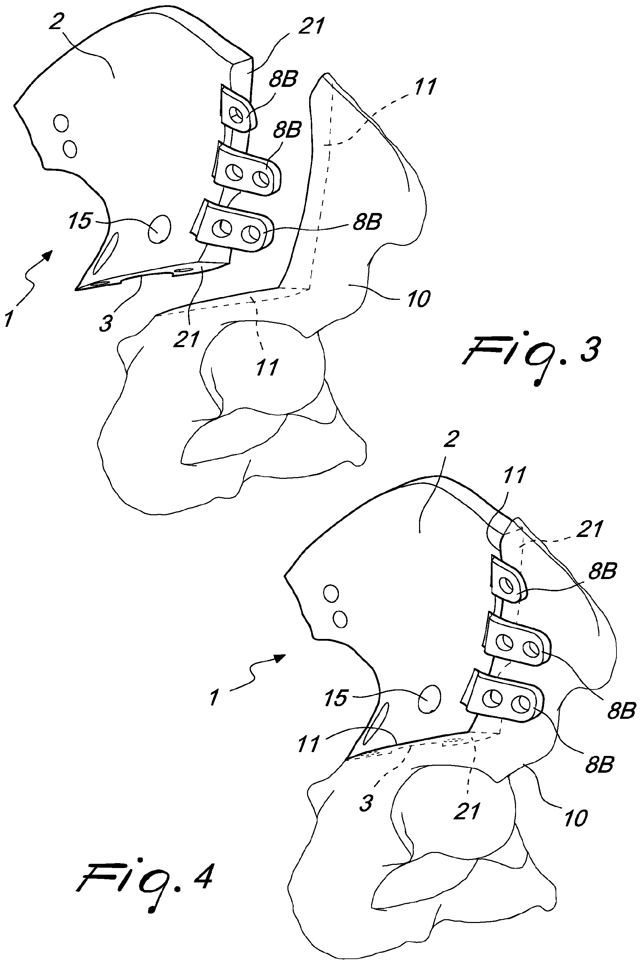

Figure 3 is an exploded perspective view, seen from a second angle of view, of the prosthesis in Figures 1 and 2;
Figure 4 is a perspective view of the prosthesis in Figure 3 positioned on the bone of the pelvis;

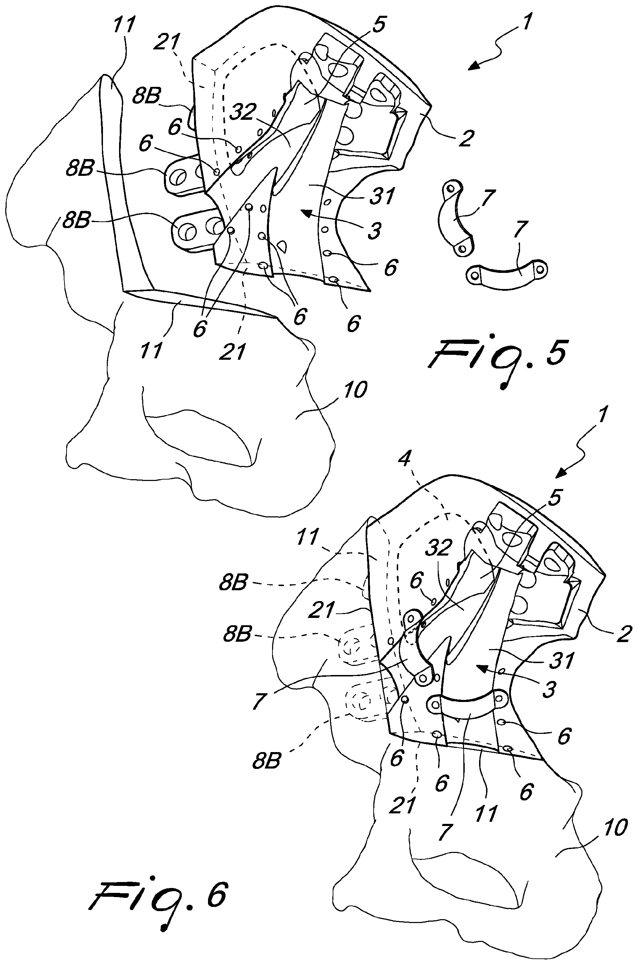

Figure 5 is an exploded perspective view, seen from a third angle of view, of the prosthesis in Figures 1 and 2;
Figure 6 is a perspective view of the prosthesis in Figure 5 positioned on the bone of the pelvis;

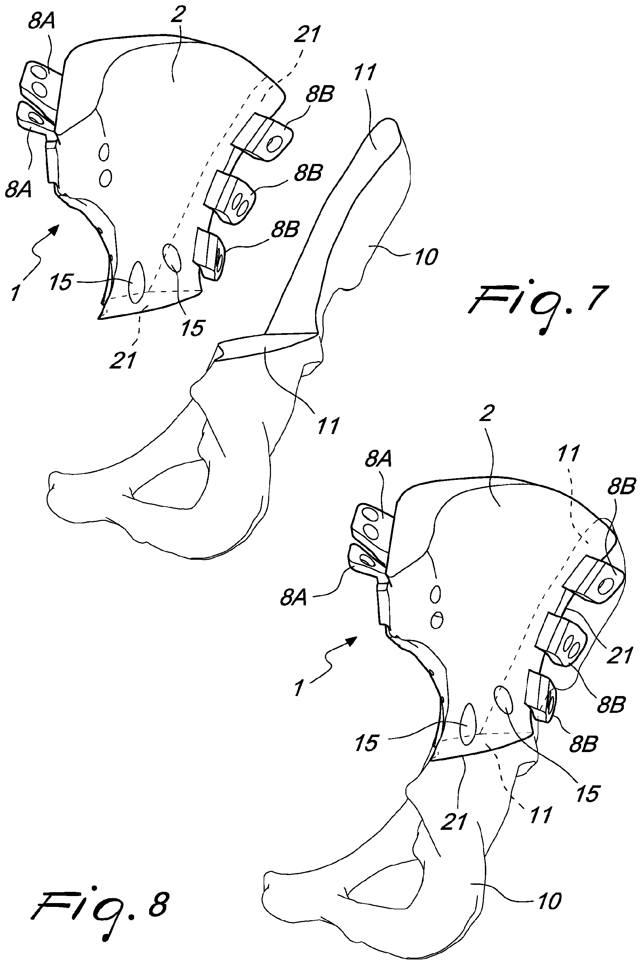

Figure 7 is an exploded perspective view, seen from a fourth angle of view, of the prosthesis in Figures 1 and 2;
Figure 8 is a perspective view of the prosthesis in Figure 7 positioned on the bone of the pelvis;

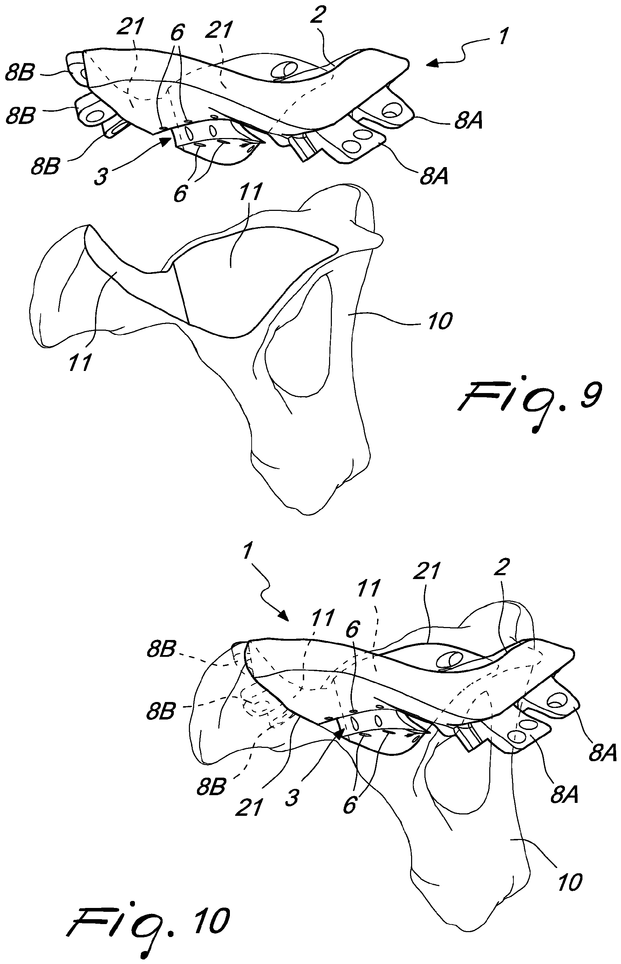

Figure 9 is an exploded perspective view, seen from a third angle of view, of the prosthesis in Figures 1 and 2;
Figure 10 is a perspective view of the prosthesis in Figure 9 positioned on the bone of the pelvis.

With reference to the figures, the prosthesis, generally designated by the reference numeral 1, comprises a prosthesis body 2 which is adapted to be coupled to a bone 11; the prosthesis body 2 comprises, to this end, at least one fixing wing 8A, 8B which protrudes externally therefrom for screw fixing on the bone 10.

The accompanying figures show, by way of non-limiting example, a hemipelvic prosthesis 1; in other embodiments the prosthesis according to the invention is intended to reconstruct other portions of bone removed during osteotomy operations and not shown here for brevity.

Preferably the prosthesis 1 is made of metal, in particular titanium, by way of a technology known in the state of the art as EBM (Electron Beam Melting), in which, briefly, the body 2 is produced via EBM of a metal dust (preferably titanium).

According to the invention, advantageously, at least one channel 3 is open onto the prosthesis body 2 and is designed to receive an insert or a graft, such as for example bone tissue, the channel 3 extending up to perimetric edges 21 of the body 2.

In more general terms, the channel 3 is designed, in use, to contain a vascularized bone graft in the form of a vascularized autologous bone graft and/or a bone graft consisting of a bone allograft (for example comprising bone fragments) and/or a vascularized fascia or a muscle, and/or a bone substitute with stem cells and/or recombinant cells and/or a device with an antimicrobial capability or function.

The prosthesis 1 also optionally comprises at least one bridge element 7 which is mounted transversely to the channel 3 and is designed to retain in position the material accommodated in the channel 3, as will be better described below.

In the embodiment shown, the bridge element 7 has an extension such as to allow only a partial covering of the channel 3; in other embodiments not shown, however, the bridge element 7 extends so as to cover the channel 3 almost completely, with a configuration that therefore becomes tubular when mounted.

For the purposes of the present description, the case is considered (for the purposes of non-limiting example) in which the prosthesis 1 is a hemipelvic prosthesis and the material contained in the channel 3 is an autologous portion of vascularized fibula.

Preferably the perimetric edges 21 of the body 2 between which the channel 3 extends are both perimetric edges for connecting the prosthesis to the bone 10 along respective osteotomy planes.

In the embodiment shown, the osteotomy planes are genuine planes in the geometric sense and, as a consequence, the edges 21 of the prosthesis 1 are correspondingly flat, since they extend on a plane.

In other embodiments, the edges 21 may not be flat, and instead have different shapes (e.g. slightly concave or convex or more generally curved), depending substantially on the osteotomy carried out.

Optionally, according to a preferred solution, the prosthesis 1 comprises a pocket 4 which extends inside the body 2 and is provided with a mouth 5 which is open onto the channel 3.

In some embodiments there are many mouths 5 of the pocket 3; likewise, in other embodiments there is a plurality (two or more) of pockets inside the body 2 and open onto the channel 3.

The pocket 4, similarly to the channel 3, is designed, in use, to contain a vascularized bone graft in the form of a vascularized autologous bone graft and/or a bone graft consisting of a bone allograft (for example comprising bone fragments) and/or a vascularized fascia or a muscle, and/or a bone substitute with stem cells and/or recombinant cells and/or a device with an antimicrobial capability or function.

In order to improve the integration of the prosthesis 1 with the bone 10, the perimetric edges 21 of the body 2 have a fine-mesh trabecular structure, preferably a mesh with a size of 700 µm.

For the same reason, optionally, at least one, preferably all, of the walls that delimit the channel 3, a face of the bridge element 7 directed toward the channel 3, or the walls that delimit the pocket 4 (if the latter is present), have a fine-mesh trabecular structure, preferably a mesh with a size of 700 µm and therefore preferably substantially equal to the structure of the perimetric edges 21 mentioned previously.

The bridge elements 7 are screw-affixed on the body 2; to this end the latter comprises a plurality of seats for screws 6 which are open in the body 2 at opposite edges of that channel 3.

There are also optionally further holes on the body 2, for example holes for reattaching capsulo-ligamentous structures to the prosthesis, for example fixing holes which are preferably adapted to receive surgical stitches which are resorbable or non-resorbable and are configured for fixing soft tissues and/or muscle fasciae to the prosthesis.

In the mounted condition, the screws are screwed in the seats 6 and fasten the bridge element 7 to the body, transversely to the channel 3; the presence of a plurality of holes 6 enables an excellent positioning of the bridge element 7, which can be done even during the actual operation to implant the prosthesis 1, without it being necessary to first choose the best position; this enables a better adaptation of the prosthesis to the physiological requirements of each patient.

In the embodiment shown, which relates to a hemipelvic prosthesis 1, the channel 3 comprises a first channel portion 31 and a second channel portion 32 which converge toward each other to form a V.

In the operating condition of the prosthesis 1, a respective bridge element 7 is mounted at each portion 31, 32 of the channel 3.

Preferably the bridge elements 7 are made of the same material as the body 2 of the prosthesis 1.

The body 2 also preferably comprises one or more holes 15 for accommodating bone screws (not shown) which are designed to engage directly on the bone 10 in order to anchor the prosthesis 1 to the latter.

Below is a method of implanting the prosthesis 1 described above.

Such method entails first of all the step:
a- of executing a computerized tomography (CT) of the bone segment of the patient,
which is followed by a subsequent step:
b- of choosing the osteotomy planes or lines as a function of the results of the previous step.

After step b comes a third step:
c- of identifying the measurements and shapes of the prosthesis 1 and of determining the anchor points thereof with the remaining bone 10 by way of screws and/or bars.

Simultaneously with or immediately after the determination in step c-, the method entails the step:
d- of measuring the diameter of the vascularized fibula segment which, for autologous bone tissue, will be inserted into the channel 3 of the prosthesis 1; this step is then followed by a step:
e- of determining the measurements (e.g. width, depth with respect to the surface of the body 2) of the channel 3, which are chosen so that the channel 3 is wider than the diameter of the vascularized fibula segment that will be inserted into the channel 3;

Once dimensions and the characteristics of the prosthesis 1 are chosen, the method entails the step:
f- of making the prosthesis 1 from titanium by way of EBS.

The method then also entails the step:
g- of taking an autologous vascularized bone graft segment from the patient;
   and the step:
h- of inserting the vascularized bone graft into the channel 3 and fixing it to the prosthesis 1 at least by way of the bridge elements 7 and/or by way of stitches and/or by way of biological bands.

The vascularized bone graft thus comes into contact with the autologous restorative vascular tissue and with the stem cells and the restorative cells present in the autologous biological liquids.

In this manner the surface of the channel 3 of the prosthesis 1 is in contact with biological fluids, restorative tissues, blood and stem cells that enable a better and more rapid integration with the existing bone and with the prosthesis. The vascularized bone of the graft in fact colonizes the trabecular titanium, filling it with living bone, capable of transforming even the mechanical properties of the prosthesis 1 which thus becomes a genuine "biological prosthesis".

If it is decided to use a single vascularized bone graft, the channel 3 will be a single channel, while if it is decided to use two vascularized bone grafts, then the channel 3 will be subdivided into the two channel portions 31, 32 as previously described, in each of which a corresponding vascularized bone graft is inserted.

The vascularized bone graft, preferably inherently autologous, can be selected from the group comprising: fibula, iliac crest, or other vascularized bones according to requirements and to the experience of the surgeon.

As an alternative to the vascularized autologous bone graft in some embodiments (single or in two portions 31 and 32) the method also entails accommodating in the channel 3, and/or in the pocket 4, a bone graft consisting of a bone allograft (for example comprising bone fragments) and/or a vascularized fascia or a muscle, and/or a bone substitute with stem cells and/or recombinant cells and/or a device with an antimicrobial capability or function.

If the vascularized bone graft is an autologous bone graft, then during the step h the fixing is carried out leaving the vascularized bone graft protrude from the channel, beyond the osteotomy line, so as to favor the integration between the prosthesis 1 and the remaining bone 10.

In some embodiments, several mutually different elements of the list just indicated are accommodated in the channel 3 and/or in the pocket 4.

Optionally, the step h above also includes fixing the vascularized graft to the prosthesis 1 by way of a biological band, for example a rotatory or free fascial or muscular strip and/or a mesh and/or one or more screws: this makes it possible to prevent micro-movements between the graft and the prosthesis 1 and the formation of secondary fibrous scar tissue in the interspace between the vascularized graft and the walls of the channel 3, which would impede the formation of living bone.

The method also entails the following step of:
i- executing at least one surgical cutting guide, configured to determine the cutting planes of an osteotomy as a function of the use of the prosthesis 1 having the characteristics identified above.

After the bone resection by way of the cutting guide, the remaining surgical margin is well exposed, so as to adapt anatomically to the prosthesis 1.

During implantation of the prosthesis 1, there is the step of:
j- fixing the prosthesis 1 to the bone 10 by way of surgical screws and/or bars; during this step, the length of the screws or of the bars can optionally be checked by way of fluoroscopy.

Preferably, for a free vascularized graft inside the groove, the micro-surgeon must suture the vessels of the graft to the receiving vessels of the patient.

Preferably, if filling the channel 3 with homologous bone fragments or bone substitutes or antimicrobial agents, the micro-surgeon can close the channel 3 with an autologous band or muscle, rotated or taken with vascular peduncle; this creates a "biological chamber" around the titanium prosthesis, rich in substrates and restorative living tissue.

In practice it has been found that the prosthesis 1, according to the present invention, achieves the intended aim and objects in that the vascularized bone tissue accommodated in the channel 3 extends between the prosthesis 1 and the bone 10, passing through the osteotomy planes 11 to the advantage of the integration between bone 10 and prosthesis 1. This makes it possible to improve the mechanical strength characteristics of the implant (bone/prosthesis assembly) because the prosthesis is deeply integrated with the bone, with considerable improvements in terms of bending and torsional strength.

The possibility of bone fusion through the osteotomy plane or line is also improved, with consequent improvements in overall terms of stability of the implant.

The prosthesis thus conceived is susceptible of numerous modifications and variations, all of which are within the scope of the appended claims.

Moreover, all the details may be substituted by other, technically equivalent elements.

In practice the materials employed, provided they are compatible with the specific use, and the contingent dimensions and shapes, may be any according to requirements.

The disclosures in Italian Patent Application No. 102018000006534 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A bone prosthesis (1), comprising a prosthesis body (2) which is adapted to be coupled to a bone (10) and which comprises, for this purpose, at least one fixing wing (8A, 8B) which protrudes externally from said body (2) for screw fixing on said bone (10),
wherein
at least one channel (3) is open onto the prosthesis body (2) and is designed to receive a graft, preferably an autologous vascularized bone tissue and/or bank bone and/or bone substitutes and/or stem cells and/or fasciae and/or muscles and/or a device with an antimicrobial capability or function, said channel (3) extending up to perimetric edges (21) of the body (2), **characterized in that** it further comprises a bridge element (7) which is mounted transversely to said channel (3).

2. The prosthesis (1) according to claim 1, **characterized in that** said perimetric edges (21) of the body (2) are perimetric edges for connecting the prosthesis to said bone (10) along respective osteotomy planes.

3. The prosthesis (1) according to claim 1, **characterized in that** said perimetric edges (21) of the body (2) have a fine-mesh trabecular structure, preferably a mesh with a size of 700 µm.

4. The prosthesis (1) according to one or more of the preceding claims, **characterized in that** it comprises a pocket (4) which extends inside the body (2) and is provided at least with one mouth (5) which is open onto said channel (3).

5. The prosthesis (1) according to one or more of the preceding claims, wherein at least one, preferably all, of walls of said channel (3), a face of said bridge element (7) directed toward said channel (3), or walls of said pocket (4), when present, have a fine-mesh trabecular structure, preferably a mesh with a size of 700 µm.

6. The prosthesis (1) according to one or more of the preceding claims, **characterized in that** it comprises a plurality of seats for screws (6) which are open in the body (2) at opposite edges of said channel (3), for screw fixing said at least one bridge element (7) transversely to said channel (3).

7. The prosthesis (1) according to one or more of the preceding claims, **characterized in that** it further comprises fixing holes which are preferably adapted to receive surgical stitches which are resorbable or non-resorbable and are configured for fixing soft tissues and/or muscle fasciae to the prosthesis.

8. The prosthesis (1) according to one or more of the preceding claims, **characterized in that** said channel (3) comprises a first channel portion (31) and a second channel portion (32) which converge toward each other, a respective bridge element (7) being mounted at each portion (31, 32).

9. The prosthesis (1) according to one or more of the preceding claims, **characterized in that** said at least one bridge element (7) extends substantially along the entire length of the channel (3).

## Patentansprüche

1. Eine Knochenprothese (1), die einen Prothesenkörper (2) umfasst, der ausgebildet ist, um mit einem Knochen (10) verbunden zu werden, und der zu diesem Zweck mindestens einen Befestigungsflügel (8A, 8B) umfasst, welcher zur Verschraubung an dem Knochen (10) außen aus dem Körper (2) herausragt,
wobei
mindestens ein Kanal (3) zum Prothesenkörper (2) hin offen ist und dazu dient, ein Transplantat, vorzugsweise ein autologes vaskularisiertes Knochengewebe und/oder Bankknochen und/oder Knochenersatz und/oder Stammzellen und/oder Faszien und/oder Muskeln und/oder eine Vorrichtung mit antimikrobieller Fähigkeit oder Funktion aufzunehmen, wobei der Kanal (3) sich bis zu Umfangsrändern (21) des Körpers (2) erstreckt; **dadurch gekennzeichnet, dass** sie weiter ein Brückenelement (7) umfasst, das quer zu dem Kanal (3) montiert ist.

2. Die Prothese (1) gemäß Anspruch 1, **dadurch gekennzeichnet, dass** die Umfangsränder (21) des Körpers (2) Umfangsränder zur Verbindung der Prothese mit dem Knochen (10) entlang entsprechenden Osteotomieebenen sind.

3. Die Prothese (1) gemäß Anspruch 1, **dadurch gekennzeichnet, dass** die Umfangsränder (21) des Körpers (2) eine feinmaschige trabekuläre Struktur haben, vorzugsweise ein Trabekelwerk mit einer Größe von 700 µm.

4. Die Prothese (1) gemäß einem oder mehreren der obigen Ansprüche, **dadurch gekennzeichnet, dass** sie eine Tasche (4) umfasst, die sich innerhalb des Körpers (2) erstreckt und mindestens mit einer Öffnung (5) zu dem Kanal (3) hin versehen ist.

5. Die Prothese (1) gemäß einem oder mehreren der obigen Ansprüche, wobei mindestens eine, vorzugsweise alle, Wände des Kanals (3), eine Seite des Brückenelements (7), die dem Kanal (3) zugewandt ist, oder Wände der Tasche (4), soweit vorhanden, eine feinmaschige trabekuläre Struktur haben, vorzugsweise ein Trabekelwerk mit einer Größe von 700 µm.

6. Die Prothese (1) gemäß einem oder mehreren der obigen Ansprüche, **dadurch gekennzeichnet, dass** sie eine Vielzahl von Sitzen für Schrauben (6) umfasst, die im Körper (2) an gegenüberliegenden Rändern des Kanals (3) offen sind, zur Verschraubung des mindestens einen Brückenelements (7) quer zu dem Kanal (3).

7. Die Prothese (1) gemäß einem oder mehreren der obigen Ansprüche, **dadurch gekennzeichnet, dass** sie weiter Befestigungslöcher umfasst, die vorzugsweise ausgebildet sind, um chirurgische Fäden aufzunehmen, die resorbierbar oder nicht resorbierbar und zur Befestigung von Weichgewebe und/oder Muskelfaszien an der Prothese ausgebildet sind.

8. Die Prothese (1) gemäß einem oder mehreren der obigen Ansprüche, **dadurch gekennzeichnet, dass** der Kanal (3) einen ersten Kanalabschnitt (31) und einen zweiten Kanalabschnitt (32) umfasst, die zusammenlaufen, wobei ein entsprechendes Brückenelement (7) an jedem Abschnitt (31, 32) angebracht ist.

9. Die Prothese (1) gemäß einem oder mehreren der obigen Ansprüche, **dadurch gekennzeichnet, dass** das mindestens eine Brückenelement (7) sich im Wesentlichen über die gesamte Länge des Kanals (3) erstreckt.

## Revendications

1. Prothèse osseuse (1), comportant un corps de prothèse (2) qui est adapté pour être couplé à un os (10) et qui comporte, à cet effet, au moins une aile de fixation (8A, 8B) qui fait saillie extérieurement à partir dudit corps (2) pour une fixation par vis sur ledit os (10),
dans laquelle
au moins un canal (3) est ouvert sur le corps de prothèse (2) et est conçu pour recevoir un greffon, de préférence un tissu osseux vascularisé autologue et/ou un os de banque et/ou des substituts osseux et/ou des cellules souches et/ou des facia et/ou des muscles et/ou un dispositif avec une capacité ou une fonction antimicrobienne, ledit canal (3) s'étendant jusqu'à des bords périmétriques (21) du corps (2), **caractérisée en ce qu'**elle comporte en outre un élément de pont (7) qui est monté transversalement audit canal (3).

2. **Prothèse (1) selon la revendication 1, caractérisée en ce que** lesdits bords périmétriques (21) du corps (2) sont des bords périmétriques destinés à relier la prothèse audit os (10) le long de plans d'ostéotomie respectifs.

3. Prothèse (1) selon la revendication 1, **caractérisée en ce que** lesdits bords périmétriques (21) du corps (2) ont une structure trabéculaire à mailles fines, de préférence une maille ayant une taille de 700 µm.

4. Prothèse (1) selon une ou plusieurs des revendications précédentes, **caractérisée en ce qu'**elle comporte une poche (4) qui s'étend à l'intérieur du corps (2) et est pourvue d'au moins une embouchure (5) qui est ouverte sur ledit canal (3).

5. Prothèse (1) selon une ou plusieurs des revendications précédentes, dans laquelle au moins une, de préférence la totalité, des parois dudit canal (3), une face dudit élément de pont (7) dirigée vers ledit canal (3), ou des parois de ladite poche (4), lorsque présente, ont une structure trabéculaire à mailles fines, de préférence une maille ayant une taille de 700 µm.

6. Prothèse (1) selon une ou plusieurs des revendications précédentes, **caractérisée en ce qu'**elle comporte une pluralité de logements pour des vis (6) qui sont ouverts dans le corps (2) sur des bords opposés dudit canal (3), pour une fixation par vis dudit au moins un élément de pont (7) transversalement audit canal (3).

7. Prothèse (1) selon une ou plusieurs des revendications précédentes, **caractérisée en ce qu'**elle comporte en outre des trous de fixation qui sont de préférence adaptés pour recevoir des sutures chirurgicales qui sont résorbables ou non résorbables et sont configurées pour fixer des tissus mous et/ou des facia musculaires à la prothèse.

8. Prothèse (1) selon une ou plusieurs des revendications précédentes, **caractérisée en ce que** ledit canal (3) comporte une première partie de canal (31) et une seconde partie de canal (32) qui convergent l'une vers l'autre, un élément de pont (7) respectif étant monté sur chaque partie (31, 32).

9. Prothèse (1) selon une ou plusieurs des revendications précédentes, **caractérisée en ce que** ledit au moins un élément de pont (7) s'étend pratiquement sur toute la longueur du canal (3).
